**Europäisches Patentamt**

**European Patent Office**

**Office européen des brevets**

(19)

(11) Veröffentlichungsnummer : **0 107 150 B2**

(12) # NEUE EUROPÄISCHE PATENTSCHRIFT

(45) Veröffentlichungstag der neuen Patentschrift :
**15.04.92 Patentblatt 92/16**

(51) Int. Cl.⁵ : **A61M 5/14**

(21) Anmeldenummer : **83110189.4**

(22) Anmeldetag : **12.10.83**

(54) **Vorrichtung zur Abgabe von Medikamenten.**

(30) Priorität : **18.10.82 DE 3238560**
**30.11.82 DE 3244337**

(43) Veröffentlichungstag der Anmeldung :
**02.05.84 Patentblatt 84/18**

(45) Bekanntmachung des Hinweises auf die
Patenterteilung :
**16.06.87 Patentblatt 87/25**

(45) Bekanntmachung des Hinweises auf die
Entscheidung über den Einspruch :
**15.04.92 Patentblatt 92/16**

(84) Benannte Vertragsstaaten :
**AT DE FR GB IT NL SE**

(56) Entgegenhaltungen :
**EP-A- 0 019 814**
**EP-A- 0 030 021**
**EP-A- 0 038 080**

(56) Entgegenhaltungen :
**EP-A- 0 049 522**
**EP-A- 0 060 306**
**DE-A- 2 450 271**
**DE-A- 2 524 789**
**DE-A- 2 539 055**
**DE-A- 2 629 720**
**DE-A- 2 810 707**
**DE-A- 3 208 819**
**DE-U- 7 604 939**
**US-A- 2 979 055**

(73) Patentinhaber : **SIEMENS**
**AKTIENGESELLSCHAFT**
**Wittelsbacherplatz 2**
**W-8000 München 2 (DE)**

(72) Erfinder : **Franetzki, Manfred, Dr.**
**Schleifweg 7B**
**W-8521 Uttenreuth (DE)**
Erfinder : **Prestele, Karl**
**Bismarckstrasse 21d**
**W-8520 Erlangen (DE)**

EP 0 107 150 B2

Jouve, 18, rue Saint-Denis, 75001 PARIS

## Beschreibung

Die Erfindung bezieht sich auf eine Vorrichtung nach dem Oberbegriff des Patentanspruchs 1. Eine solche Vorrichtung ist aus der EP-A-0 019 814 bekannt.

Vorrichtungen der eingangs genannten Art sind insbesondere zur kontinuierlichen Infusion von flüssigen Medikamenten, wie beispielsweise Heparin, Zytostatika, Analgetika, Insulin und anderen Hormonen, bekannt. Die Infusionsrate kann dabei konstant oder über einen längeren und evtl. sich zyklisch wiederholenden Zeitraum (z.B. Tagesprofil) vorprogrammiert sein, oder sie kann mit geeigneten Bedienelementen von Hand dem Bedarf angepaßt werden. Solche Vorrichtungen können Bettseitgeräte oder am Körper tragbare Geräte sein, wobei dann ein Katheter vom Gerät in den Körper geführt wird; sie können aber auch implantierbar sein, wie in der EP-A-0 019 814 näher beschrieben, wobei in der Regel ein extrakorporales Steuer- oder Programmiergerät zur Steuerung und/oder Programmierung der Infusionsrate benutzt wird. Derartige Geräte werden derzeit auch von verschiedenen Herstellern auf dem Markt angeboten.

Aus der EP-A-0 038 080 ist ein implantierbares Therapiegerät bekannt, welches den Einsatz oder Umfang einer vorgegebenen Therapie mit Hilfe eines Sensors für eine Körperfunktion bestimmt. In dieser Druckschrift wird die Aufgabe behandelt, eine vom Sensor gemeldete, zu treffende Therapiemaßnahme auch tatsächlich als solche zu erkennen und zu unterscheiden von einer aufgrund einer Störung einsetzenden Maßnahme der gleichen Art. Eine solche Störung kann von außen z.B. durch Überfliegen eines Flugzeuges eingestreut sein. Auf die Frage, wie die normale Therapiemaßnahme selbst abgebrochen werden kann, falls Störungen beim Patienten vorliegen, wird nicht eingegangen. Das bekannte Therapiegerät ist insbesondere als Defibrillator ausgestaltet und eingesetzt.

Die Therapieform der kontinuierlichen, gesteuerten oder programmierten Medikamenten-Infusion hat im Vergleich zu den herkömmlichen Medikamenten-Therapieformen zur oralen Medikamentengabe und zur Injektion den Vorteil, daß die Medikamentendosierung genauer ist und besser an einen zeitlich konstanten oder schwankenden Medikamentenbedarf angepaßt werden kann.

Unter Umständen ergibt sich aber auch ein Nachteil im Vergleich zu den herkömmlichen Therapieformen, und zwar dadurch, daß die Medikamentenzufuhr zum Körper nicht automatisch auf eine bestimmte Dosis begrenzt ist. Dies kann zu einer für den Patienten unter Umständen gefährlichen Überdosierung führen. Dies gilt insbesondere dann, wenn der Patient wegen Bewußtseinsstörung, Schlaf oder auch mangelnder Aufmerksamkeit bei zu hoch eingestellter Infusionsrate eine notwendige Reduzierung oder Abschaltung der Infusionsrate nicht vornehmen kann. Dies soll noch näher am Beispiel der Insulinbehandlung erläutert werden.

Die hier betrachteten Geräte für programmierte oder gesteuerte Medikamentendosierung werden nämlich beispielsweise, wie bereits erwähnt, bei der Diabetes-Therapie für die kontinuierliche Insulininfusion eingesetzt. Die Infusionsrate kann entweder über einen längeren Zeitraum vorprogrammiert werden (z.B. mit einem zyklisch sich wiederholenden 24-Stunden-Programm), oder sie kann mit geeigneten Bedienelementen vom Patienten den aktuellen Bedürfnissen angepaßt werden. Beispielsweise kann auf eine zeitlich konstante oder auch nach einem Tagesprofil programmierte Grund- oder Basalrate bei jeder Mahlzeit eine zeitlich begrenzte Zusatzrate aufgesetzt werden, welche dem bei der Verdauung von Kohlehydraten erhöhten Insulinbedarf Rechnung trägt.

Bei falscher Einschätzung oder bei unvorhergesehener Änderung des Insulinbedarfes, z.B. infolge von Krankheit, psychischer Erregung oder ungewöhnlicher körperlicher Aktivität, kann es zu einer Überdosierung von Insulin kommen, wodurch unter Umständen ein mit Bewußtseinsstörungen oder mit Bewußtlosigkeit verbundener hypoglykämischer Zustand eintritt. Derartige hypoglykämische Zustände sind besonders gefährlich, wenn sie während des Schlafes eintreten, weil dann kompensatorische Maßnahmen (Reduzierung oder gänzliche Abschaltung der Insulininfusion, Glukosezufuhr, u.a.) nicht oder nicht rechtzeitig ergriffen werden können. Es kann ein irreversibler Hirnschaden oder sogar der Tod des Patienten die Folge sein. Während bei konventioneller Injektion die in den Körper eingegebene Insulindosis durch das Spritzenvolumen begrenzt und nach einer bestimmten Zeit verbraucht ist, wird bei der kontinuierlichen Infusion die Insulinzufuhr auch bei Handlungsunfähigkeit oder Bewußtosigkeit des Patienten fortgesetzt. Darin liegt ein Nachteil von automatischen Dosiergeräten.

Dieser Nachteil ist nicht nur auf die Insulindosierung beschränkt. Er kann in ähnlicher Weise auch bei der Infusion anderer in der Dosierung kritischer Medikamente auftreten.

Aufgabe der Erfindung ist es, diesen Nachteil bei einer Vorrichtung der eingangs genannten Art auszugleichen. Speziell bei einer über eine gewisse Zeit andauernden Handlungsunfähigkeit des Patienten sollen selbsttätig Sicherheitsmaßnahmen eingeleitet werden. Dadurch soll die Benutzung eines automatisch arbeitenden Dosiergerätes bei weitgehender Sicherheit für den Patienten möglich sein.

Diese Aufgabe wird erfindungsgemäß durch die in dem Anspruch 1 und den Unteransprüchen angegebene Erfindung gelöst. Unter dem Begriff "Kontrollbetätigung" wird hier ein vom Patienten durchgeführter Bedie-

2

nungsvorgang verstanden. Der Patient kann dazu z.B. einen Knopf drücken, einen Schalter umlegen, ein Kontrollsignal im weitesten Sinne abgeben, um unter Beweis zu stellen, daß er voll handlungsfähig ist. Die Kontrollbetätigung kann dabei entweder mit einem normalerweise für den Betrieb erforderlichen Bedienungsvorgang identisch sein, z.B. wobei dann kein zusätzliches Bedienelement benötigt wird. Die Kontrollbetätigung kann aber auch durch ein zusätzliches Bedienelement ermöglicht werden, z.B. bei Geräten mit konstanten oder zyklisch vorprogrammierten Infusionsraten, welche keine regelmäßigen Bedienvorgänge zum Zwecke der Ratensteuerung erfordern. Die Kontrollbetätigung wird vom Dosiergerät registriert, und diese Registrierung wird zur Unterdrückung einer sonst erfolgenden Sicherheitsmaßnahme nämlich der Umschaltung des Dosiergerätes auf die reduzierte Abgaberate verwendet.

Durch die Erfindung ergibt sich die Möglichkeit, eine andauernde Überdosierung auch bei automatischen Medikamentenabgabegeräten zu verhindern. Die eingangs genannten Vorteile der automatischen Medikamentendosierung im Vergleich zu den konventionellen Verabreichungsformen bleiben dabei voll erhalten.

Der Erfindungsgedanke kann in drei vorteilhaften Ausführungsformen praktisch realisiert werden:

a) Nach Ausführung einer vom Patienten durchgeführten Kontrollbetätigung wird die programmgemäße Infusion für eine begrenzte Zeit freigegeben. Diese Zeit, die entweder fest vorgegeben oder auch einstellbar ist, ist hier mit "Karenzzeit" bezeichnet. Wenn vor Ablauf der Karenzzeit keine weitere Kontrollbetätigung erfolgt, wird automatisch die erwähnte Sicherheitsmaßnahme ausgelöst.

b) Nach Ausführung einer vom Patienten durchgeführten Kontrollbetätigung wird die programmgemäße Infusion für eine begrenzte (maximale) Dosis freigegeben. Diese Dosis, die entweder fest vorgegeben oder auch einstellbar ist, ist hier mit "Karenzdosis" bezeichnet. Wenn vor Erreichen der Karenzdosis keine weitere Kontrollbetätigung erfolgt, wird automatisch die erwähnte Sicherheitsmaßnahme ausgelöst.

c) Indem ein einstellbarer oder programmierbarer Zeitgeber vorgesehen ist, der den Zähler zu einem an die Uhrzeit gebundenen Zeitpunkt startet, lassen sich uhrzeitabhängige Erwartungsintervalle, im folgenden "Karenzintervalle" genannt, vorgeben, die z.B. an die täglichen Lebensgewohnheiten oder Bedürfnisse des Patienten angepaßt sind. Wenn innerhalb eines ersten Karenzintervalles die vorgesehene Kontrollbetätigung erfolgt, wird die programmgemäße Infusion bis zum Ablauf des nächsten oder zweiten Karenzintervalles freigegeben. Unterbleibt dagegen die vorgesehene Kontrollbetätigung, so wird nach Ablauf des ersten Karenzintervalles automatisch die erwähnte Sicherheitsmaßnahme ausgelöst.

Die Ausführungsformen a), b) und c) können auch miteinander kombiniert werden.

In Weiterbildung der Erfindung ist ein Signalgeber vorgesehen, der gleichzeitig mit beginn eines vorprogrammierten Karenzintervalles aktivierbar ist, wodurch der Patient beispielsweise an Mahlzeiten und die dafür vorgesehenen Bedienungsvorgänge erinnert wird. Dadurch läßt sich erreichen, daß im vorprogrammierten Zeitpunkt, welcher den Beginn des Karenzintervalles darstellt, beispielsweise für Frühstück, Mittagessen oder Abendessen, sofort ein Signal (Erinnerungssignal) abgegeben wird, das den Patienten zum Bedienungsvorgang auffordert. Dieser Bedienungsvorgang kann speziell bei der automatisierten, abrufgesteuerten Insulinverabreichung durch Betätigung des Abrufschalters für die Zusatzinsulindosis vor Beginn der Nahrungsaufnahme ausgelöst werden.

Wenn trotz des Erinnerungssignals das Karenzintervall abläuft, ohne daß die vorgesehene Kontrollbetätigung erfolgt, wird eine Sicherheitsschaltung zum Schutz des Patienten wirksam. Es ist zweckmäßig, wenn der Signalgeber Signale abgibt, die zu Beginn und nach Ablauf des Karenzintervalles unterschiedlich sind. Beispielsweise können zunächst einzelne oder periodische akustische Signale als Aufforderung für den Patienten erzeugt werden, während nach Ablauf des Karenzintervalles ein andauerndes und eindringlicheres Alarmsignal abgegeben wird, um gegebenenfalls auch die Umgebung des Patienten aufmerksam zu machen. Der Signalgeber und der Alarmgeber können dabei eine einzige Einheit bilden, wobei sich die unterschiedlichen Signale durch Lautstärke, Folgefrequenz und/oder andere Parameter unterscheiden lassen.

Weitere Einzelheiten und Vorteile der Erfindung ergeben sich aus der nachfolgenden Figurenbeschreibung von Ausführungsbeispielen anhand der Zeichnungen. Es zeigen:

Figur 1 einen Patienten mit einem implantierten, fernprogrammierbaren Medikamentendosiergerät,

Figur 2 den Verlauf eines Tagesinfusionsprofiles bei abrufgesteuerter Insulindosierung,

Figur 3 ein erstes Ausführungsbeispiel einer Sicherheitsschaltung für ein Dosiergerät mit Karenzzeitgeber oder Karenzintervallgeber,

Figur 4 ein zweites Ausführungsbeispiel einer Sicherheitsschaltung mit Karenzdosisgeber und

Figur 5 ein drittes Ausführungsbeispiel einer Sicherheitsschaltung mit Karenzintervallgeber und zusätzlichem Alarmgeber.

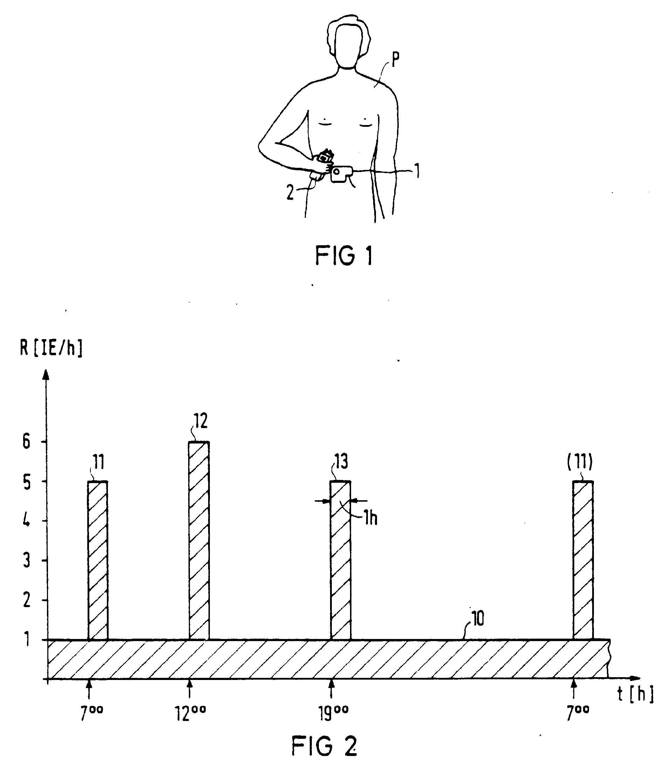

In Figur 1 ist ein Patient P mit einem externen Programmier- oder Steuergerät 2 und einem abdominal implantierten Medikamentendosiergerät 1 dargestellt. Solche Dosiergeräte werden beispielsweise in der DE-OS 29 20 976 beschrieben. Natürlich können derartige Dosiergeräte auch extrakorporal eingesetzt und durch einen Katheter mit dem Patientenkörper verbunden werden. Das Steuergerät 2 kann dann mit dem Dosiergerät 1 zu einer Funktionseinheit zusammengefaßt sein.

Figur 2 zeigt das Beispiel eines Infusionsprofiles, welches für die sogenannte abrufgesteuerte Insulindosierung in der Diabetes-Therapie typisch ist. Auf der Abszisse ist die Zeit t und auf der Ordinate die Infusionsrate R aufgetragen. Es ist eine 24-Stunden-Periode dargestellt, die von 7 Uhr morgens bis 7 Uhr morgens reicht. Mit 10 ist eine konstante Basalrate der Infusion bezeichnet, welche beispielsweise eine Internationale Insulineinheit pro Stunde (1 IE/h) beträgt. Auf die Basalrate 10 können zu den Mahlzeiten Zusatzraten 11, 12 und 13 aufgesetzt werden, welche beispielsweise auf den Kohlenhydratgehalt der jeweiligen Mahlzeit abgestimmt sind. Die schraffierte Fläche stellt die gesamt verabreichte Insulindosis dar.

Zur Aktivierung der Zusatzraten muß vom Patienten ein entsprechender Bedienungsvorgang durch Betätigung z.B. eines Schalters ausgeführt werden, welcher Vorgang in Figur 2 unten jeweils durch Pfeile symbolisiert ist. Im dargestellten Beispiel erfolgen diese Bedienungsvorgänge um 7.00 Uhr, 12.00 Uhr und 19.00 Uhr. Je nach Ausführungsform der eingesetzten Sicherheitsschaltung wären für dieses Beispiel eine Karenzzeit von ca. 13 Stunden, eine Karenzdosis von ca. 20 Insulineinheiten oder Karenzintervalle von 6 bis 8 Uhr, von 11 bis 13 Uhr und von 18 bis 20 Uhr angemessen. Im dargestellten Beispiel würde man mit den angegebenen Karenzintervallen tagsüber eine schärfere Limitierung erreichen als mit Karenzzeit oder -dosis, weil letztere wegen der langen Zeit zwischen Abendmahlzeit und Frühstück relativ groß gewählt werden muß. Eine engere Limitierung der Karenzdosis wäre möglich, wenn nur die Basalrate für die Integration der Karenzdosis berücksichtigt würde. Im Beispiel nach Figur 2 wäre dann eine Karenzdosis von ca. 13 Insulineinheiten geeignet.

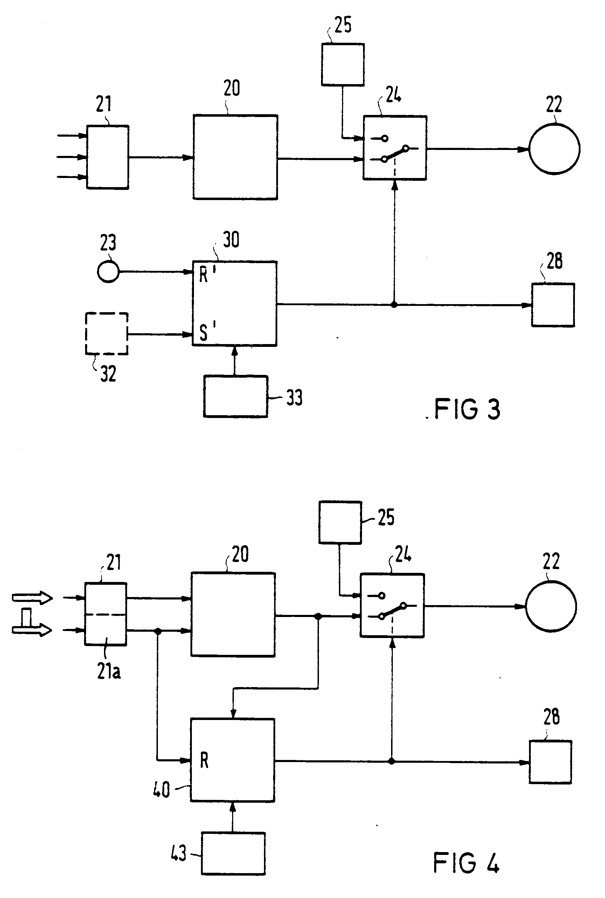

Figur 3 zeigt schematisch ein Ausführungsbeispiel einer Sicherheitsschaltung mit einem Karenzzeitgeber. Im normalen Betrieb werden von einer Ratengeberschaltung 20, welcher eine Eingabe-Einheit 21 mit Eingabe- oder Bedienelementen zugeordnet ist, nach einem vorgegebenen oder einstellbaren Infusionsprogramm Steuersignale zur Ansteuerung der Dosierpumpe 22 erzeugt. Die Steuersignale werden über einen Umschalter 24 zur Dosierpumpe 22 oder zu zugehörigen Treibereinheiten weitergeleitet. Dieser herkömmlichen Anordnung ist ein Karenzzeitgeber 30 zugeordnet, welcher aus einem Zähler besteht, der durch Betätigung eines geeineten Bedienelementes (23) mittels eines Reset-Signals oder Rücksetzimpulses R' auf Null zurücksetzbar ist. Die Abgabe des Rücksetzimpulses R' ist eine Kontrollbetätigung. Mit der Kontrollbetätigung liefert der Patient an das Dosiergerät die Information, daß er bei Sinnen ist, daß also soweit alles in Ordnung ist. Im einfachsten Fall ist der Karenzzeitgeber 30 auf eine feste Zeit, z.B. einige Stunden, eingestellt. Verstreicht die Karenzzeit, bevor ein neues Reset-Signal R' vom Patienten ausgelöst wird, so wird vom Ausgang des Karenzzeitgebers 30 einerseits ein Alarmgeber 28 und andererseits der Umschalter 24 aktiviert. Diese Aktivierung bedeutet die Einleitung von Sicherheitsmaßnahmen. Der Umschalter 24 schaltet die Steuerleitung für die Dosierpumpe 22 auf eine Notrate (reduzierte Rate) um, welche von einem Schaltkreis 25 vorgegeben wird. Die Umschaltung auf eine ungefährlich niedrige Notrate ist sinnvoller als eine vollständige Abschaltung, weil dadurch z.B. eine Verstopfung des Infusionskatheters verhindert wird. Der aktivierte Alarmgeber 28 alarmiert den Patienten. Falls erforderlich, kann dem Karenzzeitgeber 30 ein Einstellglied 33 zur Einstellung oder Programmierung der Länge der Karenzzeit zugeordnet sein. Der Beginn der Karenzzeit ist in dieser Ausführungsform an den Beginn der letzten Kontrollbetätigung gebunden.

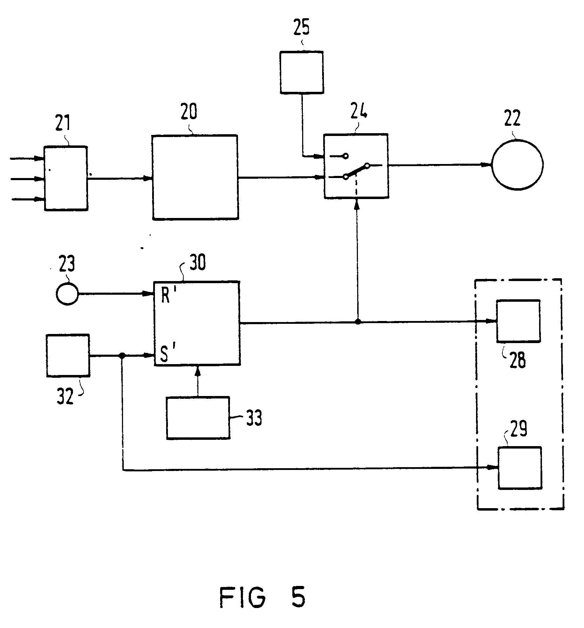

Der Karenzzeitgeber 30 kann zum Karenzintervallgeber weitergebildet werden, wenn ihm zusätzlich ein Startzeitpunktgeber 32 (gestrichelt gezeichnet) zugeordnet ist. Die Zeitmessung beginnt dann nicht nach jedem Reset-Signal R', sondern erst dann, wenn ein programmierter oder eingestellter Startimpuls S' abgegeben wird. Das Einstellglied 33 dient bei dieser Ausführungsform zur Einstellung oder Programmierung der Länge des Karenzintervalles. Die Kontrollbetätigung ist dann vom Patienten innerhalb des festgelegten Karenzintervalls vorzunehmen. Sie erfolgt über das Bedienelement 23. Dies ist bei Figur 5 noch einmal ausführlich geschildert.

Figur 4 zeigt schematisch ein Ausführungsbeispiel einer Sicherheitsschaltung mit einem Karenzdosisgeber 40. Die Bauelemente 20 bis 28 sind mit denen von Figur 3 identisch. Der Karenzzeitgeber 30 aus Figur 3 ist hier durch den Karenzdosisgeber 40 ersetzt. Mit 21a ist in der Eingabeeinheit 21 ein Abrufprogrammschalter bezeichnet, der zusätzlich zur vorhandenen Basalrate (vgl. oberer offener Pfeil) die Verabreichung einer Zusatzrate (vgl. unterer offener Pfeil) des Medikaments ermöglicht. Der Rücksetzimpuls R' für den Karenzdosisgeber 40 wird bei dieser Ausführungsform durch Betätigung des zur Aktivierung der Medikamentenzusatzrate vorhandenen Abrufschalters 21a erzeugt. Der Abrufschalter 21a ist hier also zugleich Bedienelement für die Kontrollbetätigung. Der Karenzdosisgeber 40 beginnt nach jedem Reset-Signal R' mit der Integration der von der Ratengeberschaltung 20 abgegebenen Steuersignale. Diese Integration bedeutet beispielsweise eine Impulszählung, falls die Ratensteuerung als Impulsfrequenzsteuerung ausgelegt ist. Wird eine eingestellte maximale Grenzdosis erreicht, bevor ein neues Reset-Signal R' erzeugt wird, so werden vom Ausgang des Karenzdosisgebers 40 der Alarmgeber 28 und der Umschalter 24 aktiviert, also Sicherheitsmaßnahmen eingeleitet. Dem Karenzdosisgeber 40 kann ein zusätzliches Einstellglied 43 zur Einstellung oder Programmierung der Karenzdosis zugeordnet sein.

Zur Impulszählung können Karenzzeit-, Karenzintervall- oder Karenzdosisgeber übliche rücksetzbare Zäh-

EP 0 107 150 B2

ler aufweisen, denen einstellbare oder programmierbare Zeitgeber zugeordnet sind.

Figur 5 ist weitgehend wie die Figur 3 aufgebaut. Wie bei Figur 3 besteht der Karenzintervallgeber 30 aus einem Zähler, der durch Betätigung eines geeigneten Bedienelementes, das mit 23 bezeichnet ist, mittels eines Rücksetzsignals R' auf Null zurücksetzbar ist. Das Bedienelement 23 dient zur Ausführung der Kontrollbetätigung. Vom programmierbaren Startzeitpunktgeber 32 wird ein Uhrzeitgebundenes Startsignal S' für den Zähler erzeugt. Speziell für die Insulinverabreichung im Rahmen der Diabetes-Therapie können die Startzeitpunkte beispielsweise mit einer gewissen Vorhaltezeit auf die üblichen Essenszeiten eingestellt werden. Bei Erreichen einer solchen Uhrzeit, d.h. bei Auslösen des Starts für den Zähler zum Aufzählen des eingestellten Karenzintervalles, wird ein zusätzlich vorhandener Signalgeber 29 aktiviert. Dieser Signalgeber 29 erzeugt ein im wesentlichen nur für den Patienten registrierbares Signal, beispielsweise zur Erinnerung an die Einnahme der Mahlzeiten und die damit gekoppelte, eventuell vorausgehende Betätigung des Abrufschalters. Ein solches Signal kann ein Kurzzeitton sein, - wie beispielsweise von Weckuhren bekannt -, der sich periodisch wiederholt und in der Lautstärke allmählich ansteigen kann. Durch ein solches Signal (Erinnerungssignal) wird der Patient daran erinnert, einen normalerweise zu dieser Tageszeit am Dosiergerät erforderlichen Bedienungsvorgang auszuführen vorzunehmen. Verstreicht nun das Karenzintervall trotz Erinnerung des Patienten, ohne daß ein Reset-Signal R' kommt, so werden wie oben vom Ausgang des Karenzzeitgebers 30 einerseits der Alarmgeber 28 und andererseit der Umschalter 24 aktiviert. Der Umschalter 24 schaltet die Steuerleitung zu der Dosierpumpe 22 auf eine Notrate um, welche vom Schaltkreis 25 vorgegeben wird.

Der Alarmgeber 28 und der Signalgeber 29 können eine einzige Baueinheit bilden. Diese ist dann so auszulegen, daß unterscheidbare Signale geliefert werden. Insbesondere soll durch den Alarmgeber 28 ein Alarmsignal erzeugt werden, mit dem auch die Umgebung des Patienten aufmerksam gemacht wird. Dies kann dann wichtig sein, wenn der Patient selbst handlungsunfähig ist.

Es versteht sich, daß die oben beschriebenen Sicherheitsschaltungen mit alternativen Karenzzeitgebern oder Karenzdosisgebern auch zu einer Funktionseinheit kombiniert werden können, z.B. in dem Sinne, daß die Sicherheitsschaltung schon dann anspricht, wenn entweder die Karenzzeit verstreicht oder die Karenzdosis abläuft, bevor ein vorgesehener Bedienungsvorgang stattfindet. Diese Funktion ermöglicht eine spezifische Anpassung an die jeweils vorliegenden Verhältnisse.

## Patentansprüche

1. Sensorlose Vorrichtung zur Abgabe von Medikamenten mit einem steuerbaren Dosiergerät (1), das eine Dosierpumpe (22) und eine die Dosierpumpe (22) nach einem vorgegebenen oder einstellbaren Infusionsprogramm steuernde Ratengeberschaltung (20) enthält, und mit einem gegebenenfalls separaten Programmiergerät (2), **dadurch gekennzeichnet**, daß die Vorrichtung einen durch Mittel (23,21a) zur Kontrollbetätigung rücksetzbaren und neu startbaren Zähler (30,40) aufweist, welcher auf eine vorgegebene Karenzzeit oder Karenzdosis zählt, daß der Zähler (30,40) nach Ablauf der vorgegebenen Karenzzeit bzw. vor vollständiger Abgabe der vorgegebenen Karenzdosis Mittel (24) zum Umschalten aktiviert, die unaktiviert von der Ratengeberschaltung (20) erzeugte Steuersignale an die Dosierpumpe (22) leiten und bei Aktivierung durch den Zähler (30,40) die Dosierpumpe (22) mit einem Schaltkreis (25) zur Vorgabe einer reduzierten Abgaberate verbinden.

2. Vorrichtung nach Anspruch 1, **dadurch gekennzeichnet**, daß für die Kontrollbetätigung ein separates Bedienelement (23) vorhanden ist.

3. Vorrichtung nach Anspruch 1 oder 2, **dadurch gekennzeichnet**, daß als Mittel für die Kontrollbetätigung ein für die Funktion des Dosiergerätes (1) bereits vorhandenes Bedienelement (21a) vorgesehen ist.

4. Vorrichtung nach Anspruch 3, wobei das Dosiergerät (1) ein Insulindosiergerät ist, welches mit konstanter Basalrate und zusätzlichen Abrufprogrammen arbeitet, **dadurch gekennzeichnet**, daß das Bedienelement der Abrufprogrammschalter (21a) des Insulindosiergerätes (1) ist.

5. Vorrichtung nach einem der vorangehenden Ansprüche, **dadurch gekennzeichnet**, dab ein einstellbarer oder programmierbarer Zeitgeber (32) vorgesehen ist, der den Zähler (30) zu einem an die Uhrzeit gebundenen Zeitpunkt startet.

6. Vorrichtung nach Anspruch 5, **dadurch gekennzeichnet**, daß ein Signalgeber (29) vorgesehen ist, der gleichzeitig mit Beginn eines vorprogrammierten Karenzintervalles aktivierbar ist.

## Claims

1. Sensor-less device for the delivery of medicaments with a controllable metering unit (1) which contains a metering pump (22) and a rate-generating circuit (20) which controls the metering pump (22) in accordance

5

with a preset or adjustable infusion program, and with an optionally separate program unit (2), characterised in that the device has a counter (30, 40) which can be reset and restarted by means (23, 21a) for control actuation and which counts to a preset restricted time or restricted dose, in that the counter (30, 40) after the preset restricted time has elapsed or before complete delivery of the preset restricted dose activates means (24) for switching over, which, unactivated, pass control signals generated by the rate-generating circuit (20) to the metering pump (22) and, on activation by the counter (30, 40), connect the metering pump (22) to a circuit (25) to preset a reduced delivery rate.

2. Device according to Claim 1, characterised in that a separate operating element (23) is present for control actuation.

3. Device according to Claim 1 or 2, characterised in that an operating element (21a) which is already present for the function of the metering unit (1) is provided as means for control actuation.

4. Device according to Claim 3, where the metering unit (1) is an insulin metering unit which operates with a constant baseline rate and additional call programs, characterised in that the operating element is the call program switch (21a) of the insulin metering unit (1).

5. Device according to any of the preceding claims, characterised in that an adjustable or programmable timer (32) is provided, which starts the counter (30) at a time tied to clock time.

6. Device according to Claim 5, characterised in that a signal generator (29) is provided, which can be activated at the same time as the start of a preprogrammed restricted interval.

## Revendications

1. Dispositif sans capteur pour administrer des médicaments, comportant un appareil de dosage commandable (1), qui contient une pompe de dosage (22) et un circuit générateur de débit (20) commandant la pompe de dosage (22) selon un programme d'injection prédéterminé ou réglable, et un appareil de programmation distinct (2), caractérisé par le fait que le dispositif comporte un compteur (30,40), qui peut être ramené à l'état initial et être redéclenché par des moyens (23,21a) pour réaliser l'actionnement de contrôle et qui compte jusqu'à un temps de carence ou une dose de carence prédéterminé, qu'après l'écoulement du temps de carence prédéterminé ou avant l'administration complète de la dose prédéterminée de carence, le compteur (30, 40) actionne des moyens de commutation (24), qui, à l'état inactivé, transmettent des signaux de commande produits par le circuit générateur de débit (20) à la pompe de dosage (22) et, lorsqu'ils sont activés par le compteur (30,40), relient la pompe de dosage (22) à un circuit (25) servant à prédéterminer un débit réduit d'administration.

2. Dispositif suivant la revendication 1, caractérisé par le fait qu'un organe de commande distinct (23) est prévu pour l'actionnement de contrôle.

3. Dispositif suivant la revendication 1 ou 2, caractérisé par le fait qu'il est prévu, comme moyen pour l'actionnement de contrôle, un organe de commande (21a) déjà présent pour le fonctionnement de l'appareil de dosage (1).

4. Dispositif suivant la revendication 3, dans lequel l'appareil de dosage (1) est un appareil de dosage d'insuline, qui fonctionne avec un débit basal constant et des programmes d'appel supplémentaires, caractérisé par le fait que l'organe de commande est le commutateur (21a) des programmes d'appels de l'appareil (1) de dosage de l'insuline.

5. Dispositif suivant l'une des revendications précédentes, caractérisé par le fait qu'il est prévu une minuterie réglable ou programmable (32), qui fait démarrer le compteur (30) à un instant lié à l'heure.

6. Dispositif suivant la revendication 5, caractérisé par le fait qu'il est prévu un générateur de signaux (29) qui peut être activé au moment où débute un intervalle de carence programmé d'avance.

FIG 1

FIG 2

FIG 3

FIG 4

FIG 5